Europäisches Patentamt

European Patent Office

Office européen des brevets

(19)

(11) Veröffentlichungsnummer: **0 173 999**
**B1**

(12) **EUROPÄISCHE PATENTSCHRIFT**

(45) Veröffentlichungstag der Patentschrift: **08.08.90**

(21) Anmeldenummer: **85111063.5**

(22) Anmeldetag: **02.09.85**

(51) Int. Cl.⁵: **A 23 J 1/20**, A 61 K 35/20, A 61 K 39/395

(54) **Verfahren zur Herstellung einer Lösung von Milch- und/oder Kolostralimmunglobulinen.**

(30) Priorität: **06.09.84 DE 3432718**

(43) Veröffentlichungstag der Anmeldung:
**12.03.86 Patentblatt 86/11**

(45) Bekanntmachung des Hinweises auf die
Patenterteilung:
**08.08.90 Patentblatt 90/32**

(84) Benannte Vertragsstaaten:
**AT BE CH DE FR GB IT LI LU NL SE**

(56) Entgegenhaltungen:
**CA-A-1 168 152**
**DE-A-1 949 059**
**US-A-4 051 235**

(73) Patentinhaber: **Biotest Pharma GmbH**
**Flughafenstrasse 4**
**D-6000 Frankfurt 71 (DE)**

(72) Erfinder: **Kothe, Norbert, Dr. Dipl. Chem.**
**Friedrich-Ebert-Strasse 21**
**D-6242 Kronberg/Ts (DE)**
Erfinder: **Dichtelmüller, Herbert, Dr.**
**Rossertstrasse 14**
**D-6231 Sulzbach/Ts (DE)**
Erfinder: **Stephan, Wolfgang, Dr. Dipl. Chem.**
**P.-Holzmann-Strasse 84**
**D-6072 Dreieich (DE)**
Erfinder: **Eichentopf, Bertram, Dr.**
**Im Lauer 7**
**D-6232 Bad Soden (DE)**

(74) Vertreter: **Wolff, Hans Joachim, Dr.jur. Dipl.-Chem. et al**
**Beil, Wolff & Beil Rechtsanwälte Postfach 80 01**
**40 Adelonstrasse 58**
**D-6230 Frankfurt am Main 80 (DE)**

Courier Press, Leamington Spa, England.

EP 0 173 999 B1

**Beschreibung**

Die Erfindung betrifft ein Verfahren zur Herstellung einer Lösung von Milch- und/oder Kolostralimmunglobulinen durch Aufarbeiten von Milch und/oder Kolostralmilch unter Ausfällen der Kaseine.

Bisherige Verfahren zur Gewinnung von Immunglobulinen aus Milch oder Kolostralmilch sind sehr umständlich. So wird bei dem gebräuchlichsten Verfahren zunächst das Fett durch Zentrifugieren aus der Milch abgetrennt. Anschließend wird durch Ansäuern das Kasein ausgefällt, das dann durch erneutes Zentrifugieren abgetrennt wird. Darauf wird der pH-Wert wieder auf den ursprünglichen Wert eingestellt und nochmals zentrifugiert, um den bei der Rückstellung des pH-Wertes entstandenen Niederschlag abzutrennen. Aus der auf diese Weise erhaltenen Molke werden die Immunglobuline unter Verdünnen mit Ammonsulfat ausgefällt. Der erhaltene Niederschlag wird durch Zentrifugieren abgetrennt und in Wasser gelöst und sterilfiltriert. Anschließend wird das Ammonsulfat durch Dialyse entfernt und die erhaltene Lösung nochmals sterilfiltriert und abgefüllt. Das gesamte Verfahren erfordert einen Zeitraum von mindestens drei Tagen.

Aus der EP—A—85 005 ist ein Verfahren zur Gewinnung von Immunglobulinen aus Kolostralmilch bekannt, bei dem Kolostrahlmilch oder vorzugsweise Molke von Kolostralmilch zunächst einer Elektrophorese unterworfen wird, und sodann die dabei gewonnene Fraktion, in der die Immunglobuline angereichert sind, durch Chromatographie über Ionenaustauschern fraktioniert wird. Im Effluat dieser Fraktionierung ist ein größerer Teil der Immunglobuline angereichert. Auch dieses Verfahren ist sehr umständlich und zeitraubend.

Aus der DE-OS-32-18-348 ist ein Verfahren zur Gewinnung von Lactoferrin und Immunglobulinen aus Milch bekannt, bei dem eine auf übliche Weise gewonnene Molke einer Adsorptionsbehandlung an einem festen Träger, vorzugsweise Siliciumdioxid, in einem schwach basischen Milieu bei einem pH-Wert über 7,5 unterworfen wird und die adsorbierten Proteine anschließend mit einer sauren Lösung eluient werden. Da die Immunglobuline bei diesem Verfahren nur teilweise absorbiert werden, ist es für die Gewinnung nur der Immunglobuline nicht besonders geeignet.

Die DE—A 1 949 059 betrifft ein Verfahren zum Filtrieren von Flüssigkeiten, z.B. Milch, und/oder Gasen. Hierbei wird ein Ultrafilter in Kombination mit einer zweiten gleichen Einheit verwendet. Die Porengrösse beträgt weniger als 0,1 µ. Wird eine proteinhaltige Lösung, wie Milch, filtriert, muss zunächst das Fett vor einer ersten Ultrafiltration abgetrennt werden, da Ultramembranfilter dieser Porengrösse keine gleichzeitige Entfernung von Fett und Kasein zulassen. Um ein Verstopfen der feinporigen Filter trotz Fettentfernung zu verhindern, wird hier ein Rückspülen des Filters im Gegenstrom vorgeschlagen.

Die US-PS 4 051 235 beschreibt die Herstellung eines Immunglobulin-Präparates aus Rinderkolostrum. Hierbei wird ebenfalls zunächst das Fett abgetrennt, das Kasein enzymatisch durch Zugabe von Rennin/CaCl$_2$ ausgefällt und anschliessend filtriert. Nach einer zweiten Fällung wird der erhaltene Überstand zentrifugiert und der erhaltenen Immunglobulin-Lösung Phenol zur Stabilisierung zugesetzt.

Die beiden letztgenannten Verfahren sind ebenfalls relativ zeitaufwendig, da das Fett und das Kasein in zwei verschiedenen Schritten aus dem Ausgangsmaterial, der Milch, abgetrennt werden müssen.

Der Erfindung liegt die Aufgabe zugrunde, ein einfaches und wirtschaftliches Verfahren zur Herstellung einer Lösung von Milch- und/oder Kolostralimmunglobulinen bereitzustellen.

Die Aufgabe wird erfindungsgemäß dadurch gelöst, daß man Milch und/oder Kolostralmilch auf einen pH-Wert von 4,0 bis 5,5 ansäuert und unter Anwendung einer Filtrationseinheit mit einer mittleren Porengröße von 0,1 bis 1,2 µm einer ersten Tangentialfiltration unterwirft und aus dem erhaltenen Filtrat durch eine zweite Tangentialfiltration unter Anwendung einer Filtrationseinheit mit einer Trenngrenze von 5000 bis 80 000 Dalten die niedermolekularen Bestandteile entfernt.

Das erfindungsgemäße Verfahren erfordert nur ein Drittel der für das bisher übliche Verfahren erforderlichen Zeit.

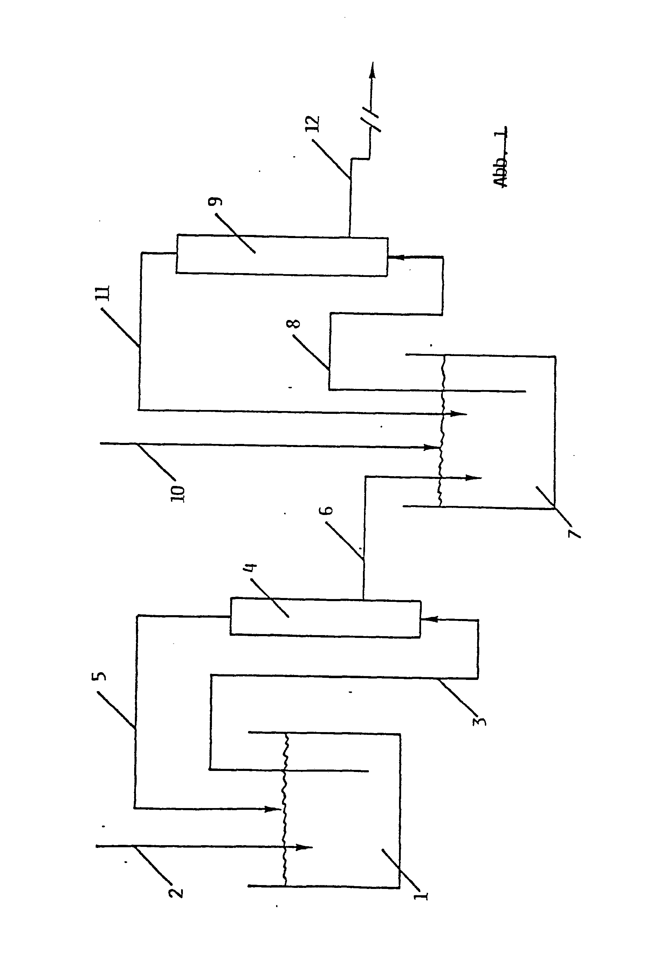

Abb. 1 zeigt ein Fließschema des erfindungsgemäßen Verfahrens. Im Behälter 1 wird die Milch und/oder Kolostralmilch auf einen pH-Wert von 4,0 bis 5,5, vorzugsweise auf einen pH-Wert von 4,8 angesäuert. Zum Ansäuern kann z.B. verdünnte Salzsäure oder ein Acetatpuffer verwendet werden. Vorzugsweise wird die Milch und/oder Kolostralmilch vor oder nach dem Ansäuren oder während des Ansäuerns über Leitung 2 mit einer 0,3 bis 1,5%igen Elektrolytlösung, insbesondere mit einer 0,9%igen Kochsalzlösung verdünnt, beispielsweise im Verhältnis 1:2. Das angesäuerte Produkt wird sodann über Leitung 3 durch eine Filtrationseinheit 4 mit einer Porengröße von 0,1 bis 1,2 µm gepumpt. Bei dieser ersten Tangentialfiltration (cross-flow-Filtration) im Bereich der Mikrofiltration, bei der vorzugsweise eine Hohlfaserpatrone mit einer Porengröße von 0,4 µm zur Diafiltration verwendet wird, wird das gefällte Kasein zurückgehalten und als Filtrat eine klare Lösung, die die Immunglobuline und niedermolekulare Bestandteile enthält, gewonnen. Hohlfaserpatronen mit mittleren Porengrößen von 0,1 bis 1,2 µm, die für diese erste Tangentialfiltration als Filtrationseinheit eingesetzt werden können, sind im Handel erhältlich. Die Patronen sind in unterschiedlichen Längen erhältlich und haben hohe Membranflächen. Je nach der Länge der verwendeten Hohlfaserpatrone wird bei der Mikrofiltration mit einem Überdruck von 0,1 bis 1 bar gearbeitet. Die Temperatur ist nicht kritisch. Man arbeitet bei 4 bis 40°C, vorzugsweise bei Raumtemperatur. Das in der Hohlfaserpatrone 4 zurückgehaltene Kasein wird über Leitung 5 in den Behälter 1 zurückgeführt. Das Volumen des Behälters 1 wird durch Zusatz von Kochsalzlösung konstant gehalten. Die erhaltene filtrierte Lösung wird über Leitung 6 in den Behälter 7 geleitet.

Aus dem Behälter 7 wird die Lösung sodann über Leitung 8 durch eine Hohlfaserpatrone 9 mit einer Trenngrenze von 5 000 bis 80 000 Dalten geleitet, in der durch eine zweite Tangentialfiltration die niedermolekularen Bestandteile aus der Immunglobulinlösung entfernt werden. Hohlfaserpatronen als Filtrationseinheit für diese zweite Tangentialfiltration im Bereich der Ultrafiltration sind ebenfalls im Handel erhältlich. Vorzugsweise wird eine Hohlfaserpatrone mit einer Trenngrenze von 10 000 Dalton verwendet. Über Leitung 10 wird Kochsalzlösung, vorzugsweise mit einer Konzentration von 0,9%, in den Behälter 7 eingeleitet. Die in der Hohlfaserpatrone 9 zurückgehaltene, gereinigte Immunglobulinlösung wird über Leitung 11 in den Behälter 7 zurückgeführt. Das die niedermolekularen Bestandteile enthaltende Filtrat wird über Leitung 12 aus der Hohlfaserpatrone 9 entfernt. Bei der zweiten Tangentialfiltration wird vorzugsweise zunächst ankonzentriert, dann diafiltriert und schließlich die Konzentration der Immunglobulinlösung durch Ankonzentration auf den gewünschten Proteingehalt, beispielsweise auf einen Proteingehalt von 5 bis 10% eingestellt. Anschließend wird die Immunglobulinlösung in bekannter Weise sterilfiltriert.

Als Ausgangsmaterial des erfindungsgemäßen Verfahrens kann Milch und/oder Kolostralmilch verwendet werden. Wenn Milch verwendet wird, sollten die trächtigen Säugetiere in bekannter Weise hyperimmunisiert werden, um die Ausbeute an spezifischen Immunglobulinen zu erhöhen.

Wenn als Ausgangsmaterial eine Kolostralmilch verwendet wird, kann auf eine aufwendige Hyperimmunisierung der trächtigen Säugetiere verzichtet werden, sofern die Kolostralmilch in den ersten Stunden nach der Geburt entnommen wurde. Überraschenderweise wurde festgestellt, daß Kolostralmilch, die kurz nach der Geburt entnommen wurde, sehr hohe Titer gegen verschiedene bakterielle und virale Antigene enthält. Verglichen mit Milch von hyperimmunisierten Tieren kann dieser Titer gegen die Antigene 50- bis 100-fach höher liegen. Ein bevorzugtes Ausgangsmaterial des erfindungsgemäßen Verfahrens ist daher eine Kolostralmilch von nicht hyperimmunisierten Säugetieren oder Humankolostralmilch, wobei eine von Rindern bis 30 Stunden nach dem Kalben und insbesondere eine bis 5 Stunden nach dem Kalben entnommene Kolostralmilch besonders bevorzugt wird.

Nach dem erfindungsgemäßen Verfahren kann beispielsweise aus 1 l Kolostralmilch etwa 1 l einer sterilfiltrierten 5%igen Immunglobulinlösung gewonnen werden, die zur Behandlung von bakteriellen und viralen Darminfektionen geeignet ist und zwar sowohl in der Humanmedizin als auch in der Veterinärmedizin. Die erhaltene Immunglobulinlösung eignet sich insbesondere zur oralen Anwendung bei Frühgeburten und allen Säuglingen mit Darmerkrankungen sowie zur intravenösen Anwendung in der Veterinärmedizin.

Die nach dem erfindungsgemäßen Verfahren erhaltene Immunglobulinlösung ist völlig klar und bei 37°C ohne stabilisierende Zusätze beständig. Ihr Lipidgehalt ist auf ewta 3% des Ausgangsmaterials verringert. Sie enthält nur etwa 25% der störenden proteolytischen Aktivität einer durch Behandlung mit β-Propiolacton und UV-Strahlen stabilisierten Immunglobulinlösung, die aus Blutserum erhalten wurde. Ihre unspezifische Komplementbindung entspricht denjenigen von bekannten, aus Blutserum erhaltenen Immunglobulinpräparaten, die mit β-Propiolacton modifiziert oder mit Enzymen behandelt wurden.

Fur eine nach dem erfindungsgemäßen Verfahren erhaltene 5%ige Immunglobulinlösung wurden beispielsweise die folgenden Antikörpertiter bestimmt:

| E. coli | 1:160 | (passive Hämagglutination) |
|---|---|---|
| Ps. aeruginosa | 1:80 | " |
| Klebsiella | 1:160 | " |
| Staphylokokken | 1:20 | " |
| Enterokokken | 1:20 | " |
| Streptokokken | 1:40 | " |
| Rotavirus | 1:32 | (Komplementbindungsreaktion) |

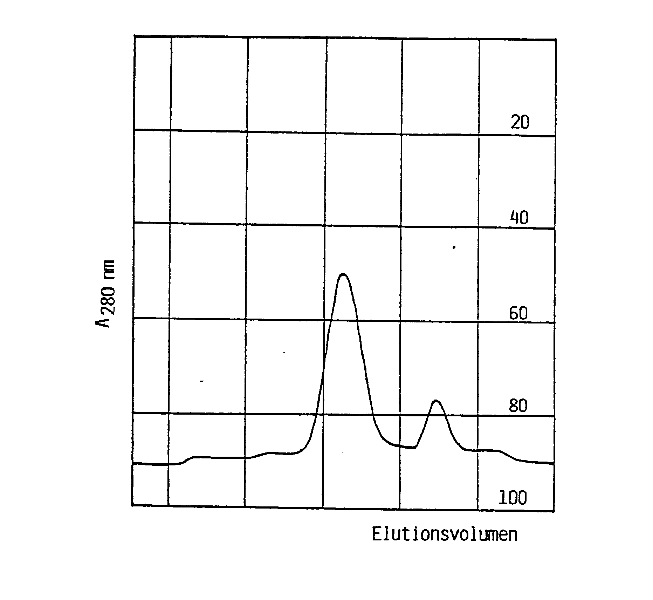

Diese Immunglobulinlösung ergab bei einer für Immunglobuline üblichen Gelchromatographie an Dextrangel das in Abb. 2 gezeigte Elutionsprofil, dem zu entnehmen ist, daß die Lösung hauptsächlich IgG neben einem geringen Anteil an Lactalbumin enthielt. Durch Immunelektrophorese wurden IgM, IgA und sekretorisches IgA nachgewiesen. Eine Elektrophorese auf Celluloseacetat-Folien ergab einen Gehalt der Feststoffe der Lösung von 77% Gammaglobulin (Immunglobulin), 1,2% Lactalbumin, 3,4% β-Globulin sowie 17% $\alpha_1$- und $\alpha_2$-Globuline.

Diese Immunglobulinlösung wurde im Infektionsversuch an der Maus auf ihre antibakterielle Wirksamkeit geprüft. Hierzu wurden Mäuse i.p. mit $1 \times 10^7$ Keimen von Ps. aeruginosa infiziert und anschließend i.p. mit 0,5 ml der Immunglobulinlösung behandelt. Eine Kontrollgruppe wurde lediglich infiziert. Als Ergebnis zeigte sich, daß alle unbehandelten Tiere 20 Stunden nach der Infektion verendet waren, während die mit der Immunglobulinlösung behandelten Tiere zu 100% geschützt waren. Diese Schutzwirkung übertraf bei weitem die einer bekannten Immunglobulinlösung gleicher Proteinkonzentration, die aus humanem Serum oder Plasma erhalten worden war.

Das erfindungsgemäße Verfahren wird durch die folgenden Beispiele erläutert:

3

## EP 0 173 999 B1

Beispiel 1

Rinderkolostralmilch, die in den ersten 5 Stunden nach dem Kalben entnommen worden war, wurde tiefgefroren. 3,75 l dieser tiefgefrorenen Kolostralmilch wurden aufgetaut. Der pH-Wert betrug 6,22 und wurde durch Ansäuern mit 220 ml 1 n HCl auf 4,8 eingestellt. Anschließend wurde die angesäuerte Kolostralmilch 30 Minuten bei 40°C und dann über Nacht bei 4°C aufbewahrt.

Die angesäuerte und gefällte Kolostralmilch wurde sodann zur Beseitigung grober Partikel über ein Transfusionsfilter in ein Vorratsgefäß gepumpt und mit 0,9%iger Kochsalzlösung auf ein Gesamtvolumen von 6 l verdünnt.

Die so erhaltene Suspension wurde einer ersten Tangentialfiltration durch zwei parallele Hohlfaserpatronen, die jeweils eine Porengröße von 0,4 µm und eine Oberfläche von 0,5 m² aufwiesen, unterworfen. Dabei wurde die Suspension im Verlauf von 4 Stunden unter Diafiltration mit 21 l einer 0,9%igen Kochsalzlösung bei einem Überdruck von 0,25 bar durch die beiden Hohlfaserpatronen gepumpt.

Noch während des Verlaufs der ersten Tangentialfiltration wurde das angefallene Diafiltrat einer zweiten Tangentialfiltration durch eine Hohlfaserpatrone mit einer Trenngrenze von 10 000 Dalten und einer Oberfläche von 0,9 m² zugeführt, wobei ein Überdruck von 1 bar angewendet wurde. In dieser Hohlfaserpatrone der zweiten Tangentialfiltration wurde die Lösung zunächst amkonzentriert, wobei das Volumen im Verlauf von 3 Stunden konstant bei 5 l gehalten wurde, anschließend diafiltriert, wobei 20 l einer 0,9%igen Kochsalzlösung verwendet werden, und dann stufenweise auf ein Volumen von 1,4 l und einen Proteingehalt von 9,7% ankonzentriert. Das die niedermolekularen Bestandteile enthaltende Filtrat wurde entfernt.

Die so erhaltene Immunglobulinlösung wurde mit 45 ml 1n NaOH auf einen pH-Wert von 7,4 eingestellt, anschließend sterilfiltriert und in Mengen von jeweils 100 ml abgefüllt.

Beispiel 2

Von einer wie in Beispiel 1 tiefgefrorenen Rinderkolostralmilch wurden 13 l aufgetaut, deren pH-Wert 6,27 betrug. Der pH-Wert wurde durch Ansäuern mit 700 ml 1n HCl auf 4,8 eingestellt. Die so erhaltene Suspension wurde 30 Minuten lang auf 40°C erwärmt und dann über Nacht bei 4°C aufbewahrt.

Anschließend wurde die Suspension zur Abtrennung grober Partikel durch ein Gazefilter aus Polyamidgaze in ein Vorratsgefäß gepumpt und dort mit 0,9%iger Kochsalzlösung auf ein Gesamtvolumen von 26 l verdünnt.

Dann erfolgte die erste Tangentialfiltration der so erhaltenen Suspension durch eine Hohlfaserpatrone mit einer Porengröße von 0,4 µm, einer Oberfläche von 3 m² und einem Faserdurchmesser von 1,2 mm durch Diafiltration unter Verwendung von 100 l einer 0,9%igen Kochsalzlösung bei einem Überdruck von 0,2 bis 0,6 bis.

Noch während des Verlaufs der ersten Tangentialfiltration wurde das angefallene Diafiltrat einer zweiten Tangentialfiltration unter Verwendung einer aus drei Hohlfaserpatronen mit jeweils einer Trenngrenze von 10 000 D und einer Oberfläche von 1,4 m² bestehenden Anlage zugeführt. In dieser Anlage wurde das Diafiltrat der ersten Tangentialfiltration zunächst ankonzentriert, sodann unter Verwendung von 100 l einer 0,9%igen Kochsalzlösung diafiltriert und schließlich auf ein Gesamtvolumen von 25 l ankonzentriert. Das die niedermolekularen Bestandteile enthaltende Filtrat wurde entfernt.

Die erhaltene Immunglobulinlösung wies einen Proteingehalt von 6% (bestimmt durch Biuret-Reaktion) auf und wurde in bekannter Weise sterilfiltriert.

Beispiel 3

Von einer wie in Beispiel 1 tiefgefrorenen Rinderkolostralmilch wurden 15 l aufgetaut, deren pH-Wert 6,20 betrug. Der pH-Wert wurde durch Ansäuren mit 1000 ml 1n HCl auf 4,82 eingestellt. Die so erhaltene Suspension wurde 30 Minuten lang auf 40°C erwärmt und dann bei 4°C mit 0,9%iger Kochsalzlösung auf ein Gesamtvolumen von 30 l verdünnt.

Die so erhaltene, verdünnte Suspension wurde zur ersten Tangentialfiltration bei einem Überdruck von 0,6 bar durch eine Hohlfaserpatrone mit einer Porengröße von 0,4 µm, einer Oberfläche von 2 m² und einem Faserdurchmesser von 1,8 mm gepumpt, wobei unter Verwendung von 150 l einer 0,9%igen Kochsalzlösung diafiltriert wurde.

Das Filtrat der ersten Tangentialfiltration, das mit einem Filtratfluß von 25 l pro Stunde anfiel, wurde zur zweiten Tangentialfiltration der in Beispiel 2 verwendeten Anlage aus drei Hohlfaserpatronen mit jeweils einer Trenngrenze von 10 000 Dalton und einer Oberfläche von 1,4 m² zugeführt. In dieser Anlage wurde zunächst eine Ankonzentrierung, bei der das Volumen konstant bei 25 l gehalten wurde, durchgeführt. Anschließend wurde unter Verwendung von 120 l einer 0,9%igen Kochsalzlösung diafiltriert, und schließlich wurde das Filtrat auf einen Proteingehalt von 7,8% ankonzentriert. Der Überdruck betrug 1,0 bis 1,7 bar. Das die niedermolekularen Bestandteile enthaltende Filtrat wurde entfernt.

Die so erhaltene Immunglobulinlösung wurde durch Zugabe von etwa 130 ml 1n NaOH auf einen pH-Wert von 7,45 eingestellt, anschließend unter Verwendung eines Mehrschichtenfilters sterilfiltriert und in Teilmengen abgefüllt.

4

EP 0 173 999 B1

Die erhaltene Immunglobulinlösung hatte die folgende Zusammensetzung:

| | |
|---|---|
| Albumin | 1,5% |
| α-Globuline | 19% |
| ß-Globuline | 3,1% |
| Immunglobuline (μ) | 77,4% |
| davon IgG ca. | 85% |
| IgM ca. | 10%. |

Sie wies keine nachweisbare antikomplementäre Aktivität auf und es wurden darin die folgenden Antikörpertiter bestimmt:

Bakterielle
Antikörper:

| | | |
|---|---|---|
| E. coli | 1:2560 | (passive Hämagglutination) |
| Ps.aeruginosa | 1:640 | „ |
| Klebsiella | 1:320 | „ |
| Staphylokokken | 1:80 | „ |
| Enterkokokken | 1:40 | „ |
| Streptokokken | 1:40 | „ |

Virale
Antikörper:

| | | |
|---|---|---|
| Varizellen | <1:10 | (Komplementbindungsreaktion) |
| Cytomegalie | <1:10 | |
| Rubella | <1:8 | |
| Herpes | 1:20 | |
| Rotavirus | 1:32 | |

## Patentansprüche

1. Verfahren zur Herstellung einer Lösung von Milch- und/oder Kolostralimmunglobulinen durch Aufarbeiten von Milch und/oder Kolostralmilch unter Ausfällen der Kaseine, dadurch gekennzeichnet, daß man Milch und/oder Kolostralmilch auf einen pH-Wert von 4,0 bis 5,5 ansäuert und unter Anwendung einer Filtrationseinheit mit einer mittelbaren Porengröße von 0,1 bis 1,2 μm einer ersten Tangentialfiltration unterwirft und aus dem erhaltenen Filtrat durch eine zweite Tangentialfiltration unter Anwendung einer Filtrationseinheit mit einer Trenngrenze von 5 000 bis 80 000 Dalton die niedermolekularen Bestandteile entfernt.

2. Verfahren nach Anspruch 1, dadurch gekennzeichnet, daß man als Ausgangsmaterial Kolostralmilch verwendet.

3. Verfahren nach Anspruch 2, dadurch gekennzeichnet, daß man als Ausgangsmaterial Kolostralmilch von nicht hyperimmunisierten Säugetieren oder Humankolostralmilch verwendet.

4. Verfahren nach Anspruch 2 oder 3, dadurch gekennzeichnet, daß man als Ausgangsmaterial eine von Rindern bis 30 Stunden nach dem Kalben entnommene Kolostralmilch verwendet.

5. Verfahren nach Anspruch 4, dadurch gekennzeichnet, daß man als Ausgangsmaterial eine von Rindern bis 5 Stunden nach dem Kalben entnommene Kolostralmilch verwendet.

6. Verfahren nach einem der Ansprüche 1 bis 5, dadurch gekennzeichnet, daß man das Ausgangsmaterial vor, nach oder während des Ansäuerns mit einer 0,3 bis 1,5%igen Elektrolytlösung verdünnt.

7. Verfahren nach einem der Ansprüche 1 bis 6, dadurch gekennzeichnet, daß man das Ausgangsmaterial auf einen pH-Wert von 4,8 ansäuert.

8. Verfahren nach einem der Ansprüche 1 bis 7, dadurch gekennzeichnet, daß man zur ersten Tangentialfiltration des angesäuerten Materials eine Hohlfaserpatrone mit einer Porengröße von 0,4 μm verwendet.

9. Verfahren nach einem der Ansprüche 1 bis 8, dadurch gekennzeichnet, daß man zur zweiten Tangentialfiltration des Filtrates eine Hohlfaserpatrone mit einer Trenngrenze von 10 000 Dalton verwendet.

10. Verfahren nach einem der Ansprüche 1 bis 9, dadurch gekennzeichnet, daß man bei der zweiten Tangentialfiltration die Konzentration der Lösung auf einen gewünschten Proteingehalt einstellt und die Lösung anschließend sterilfiltriert.

## Revendications

1. Procédé de préparation d'une solution d'immunoglobulines du lait et/ou du colostrum par traitement du lait et/ou du colostrum avec précipitation des caséines, caractérisé en ce qu'on acidifie du lait

5

et/ou du colostrm à un pH de 4,0 à 5,5 et en ce qu'on le soumet en utilisant une unité de filtration ayant une taille moyenne de pores de 0,1 à 1,2 µm à une première filtration tangentielle et en ce qu'on enlève les composants à faible poids moléculaire du filtrat obtenu par une seconde filtration tangentielle en utilisant une unité de filtration ayant une limite de séparation de 5000 à 80 000 dalton.

2. Procédé selon la revendication 1, caractérisé en ce qu'on utilise comme produit de départ du colostrum.

3. Procédé selon la revendication 2, caractérisé en ce qu'on utilise comme produit de départ du colostrum de mammifères non hyperimmunisés ou du colostrum humain.

4. Procédé selon la revendication 2 ou 3, caractérisé en ce qu'on utilise comme produit de départ un colostrum prélevé chez des bovins jusqu'à 30 heures après le vêlage.

5. Procédé selon la revendication 4, caractérisé en ce qu'on utilise comme produit de départ un colostrum prélevé chez des bovins jusqu'à 5 heures après le vêlage.

6. Procédé selon l'une des revendications 1 à 5, caractérisé en ce qu'on dilue le produit de départ avant, après ou pendant l'acidification avec une solution d'électrolyte à 0,3 à 1,5%.

7. Procédé selon l'une des revendications 1 à 6, caractérisé en ce qu'on acidifie le produit de départ à un pH de 4,8.

8. Procédé selon l'une des revendications 1 à 7, caractérisé en ce qu'on utilise pour la première filtration tangentielle de la matière, acidifiée un patron de fibres à jour ayant une taille de pores de 0,4 µm.

9. Procédé selon l'une des revendications 1 à 8, caractérisé en ce qu'on utilise pour la deuxième filtration tangentielle du filtrat un patron de fibres à jour ayant une limite de séparation de 10 000 dalton.

10. Procédé selon l'une des revendications 1 à 9, caractérisé en ce qu'on règle dans la deuxième filtration tangentielle la concentration de la solution à une teneur en protéines désirée puis en ce qu'on filtre la solution de facon stérile.

## Claims

1. Process for preparing a solution of immunoglobulins of milk and/or colostrum by the working up of milk and/or colostrum with precipitation of the caseins, characterised in that milk and/or colostrum is acidified to a pH of from 4.0 to 5.5 and subjected to a first tangential filtration by means of a filtration unit having an indirect pore size of from 0.1 to 1.2 µm and the low molecular weight constituents are removed from the resulting filtrate by a second tangential filtration using a filtration unit having a separating limit of from 5000 to 80,000 Dalton.

2. Process according to Claim 1, charaterised in that the starting material used is colostrum.

3. Process according to Claim 2, characterised in that the starting material used is colostrum of non-hyperimmunised mammals or human colostrum.

4. Process according to Claim 2 or 3, characterised in that the starting material used is a colostrum taken from cows up to 30 hours after calving.

5. Process according to Claim 4, characterised in that the starting material used is a colostrum taken from cows up to 5 hours after calving.

6. Process according to one of the Claims 1 to 5, characterized in that the starting material is diluted with a 0.3 to 1.5% electrolyte solution before, after or during the acidification.

7. Process according to one of the Claims 1 to 6, characterised in that the starting material is acidified to a pH of 4.8.

8. Process according to one of the Claims 1 to 7, characterised in that a hollow fibre cartridge having a pore size of 0.4 µm is used for the first tangential filtration of the acidified material.

9. Process according to one of the Claims 1 to 8, characterised in that a hollow fibre cartridge having a separating limit of 10,000 Dalton is used for the second tangential filtration of the filtrate.

10. Process according to one of Claims 1 to 9, characterised in that the concentration of the solution for the second tangential filtration is adjusted to a desired protein content and the solution is then filtered sterile.

Abb. 1

EP 0 173 999 B1